# DEMANDE DE BREVET EUROPEEN

(11) **EP 1 683 807 A1**
(43) Date de publication de la demande: **26.07.2006**
(21) Numéro de dépôt: 06113878.0
(22) Date de dépôt: 03.01.2003
(51) Int. Cl.: C07K 14/53, C12Q 1/68, C12N 15/11, A61K 31/7105

(54) **Produits de combinaison utilisable dans le cadre d'un traitement antitumoral comprenant un anticoprs capable d'inhiber l'activité de CSF-1**

(30) Priorité: 03.01.2002 FR 0200029
(62) Demande divisionnaire de: 03712203.3
(71) Demandeur: TRANSGENE S.A., 67000 Strasbourg (FR); Institut Curie, 75005 Paris (FR)
(72) Inventeur: Balloul, Jean-Marc, 67380, Lingolsheim (FR); Scholl, Suzy, 75010, Paris (FR); Lacoste, Jérôme, 38000, Grenoble (FR)
(74) Mandataire: Warcoin, Jacques

(57) **Abrégé**

La présente invention concerne des produits de combinaison comprenant (i) au moins une substance capable d'inhiber l'activité de CSF-1 et/ou au moins un acide nucléique, comprenant au moins une séquence codant pour une substance capable d'inhiber l'activité de CSF-1 et (ii) au moins une substance ayant une activité cytotoxique et/ou au moins un acide nucléique, comprenant au moins une séquence codant pour une substance ayant une activité cytotoxique. La présente invention concerne également des oligonucléotides capables d'inhiber l'expression de CSF-1. La présente invention est particulièrement utile dans le cadre de la mise en oeuvre d'un traitement antitumoral.

LISTE DE SEQUENCES

<110> TRANSGENE SA
      institut curie

<120> Produits de combinaison utilisable dans le cadre d'un
      traitement antitumoral.

<130> CSF1

<140>
<141>

<150> FR0200029
<151> 2002-01-03

<160> 5

<170> PatentIn Ver. 2.1

<210> 1
<211> 3939
<212> ADN
<213> Homo sapiens

<300>

<400> 1
cctgggtcct ctcggcgcca gagccgctct ccgcatccca ggacagcggt gcggccctcg 60
gccggggcgc ccactccgca gcagccagcg agccagctgc cccgtatgac cgcgccgggc 120
gccgccgggc gctgccctcc cacgacatgg ctgggctccc tgctgttgtt ggtctgtctc 180
ctggcgagca ggagtatcac cgaggaggtg tcggagtact gtagccacat gattgggagt 240
ggacacctgc agtctctgca gcggctgatt gacagtcaga tggagacctc gtgccaaatt 300
acatttgagt ttgtagacca ggaacagttg aaagatccag tgtgctacct taagaaggca 360
tttctcctgg tacaagacat aatggaggac accatgcgct tcagagataa caccgccaat 420
cccatcgcca ttgtgcagct gcaggaactc tctttgaggc tgaagagctg cttcaccaag 480
gattatgaag agcatgacaa ggcctgcgtc cgaactttct atgagacacc tctccagttg 540
ctggagaagg tcaagaatgt ctttaatgaa acaaagaatc tccttgacaa ggactggaat 600
attttcagca agaactgcaa caacagcttt gctgaatgct ccagccaaga tgtggtgacc 660
aagcctgatt gcaactgcct gtaccccaaa gccatcccta gcagtgaccc ggcctctgtc 720
tcccctcatc agcccctcgc cccctccatg gcccctgtgg ctggcttgac ctgggaggac 780
tctgagggaa ctgagggcag ctccctcttg cctggtgagc agcccctgca cacagtggat 840
ccaggcagtg ccaagcagcg gccacccagg agcacctgcc agagctttga gccgccagag 900
accccagttg tcaaggacag caccatcggt ggctcaccac agcctcgccc ctctgtcggg 960
gccttcaacc ccgggatgga ggatattctt gactctgcaa tgggcactaa ttgggtccca 1020
gaagaagcct ctggagaggc cagtgagatt cccgtacccc aagggacaga gctttccccc 1080
tccaggccag gagggggcag catgcagaca gagcccgcca gacccagcaa cttcctctca 1140
gcatcttctc cactccctgc atcagcaaag ggccaacagc cggcagatgt aactgctaca 1200
gccttgccca gggtgggccc cgtgatgccc actggccagg actggaatca caccccccag 1260
aagacagacc atccatctgc cctgctcaga gaccccccgg agccaggctc tcccaggatc 1320
tcatcactgc gcccccaggc cctcagcaac ccctccaccc tctctgctca gccacagctt 1380
tccagaagcc actcctcggg cagcgtgctg ccccttgggg agctggaggg caggaggagc 1440
accagggatc ggacgagccc cgcagagcca gaagcagcac cagcaagtga aggggcagcc 1500
aggcccctgc cccgttttaa ctccgttcct ttgactgaca caggccatga gaggcagtcc 1560
gagggatcct ccagcccgca gctccaggag tctgtcttcc acctgctggt gcccagtgtc 1620
atcctggtct tgctggctgt cggaggcctc ttgttctaca ggtggaggcg gcggagccat 1680
caagagcctc agagagcgga ttctcccttg gagcaaccag agggcagccc cctgactcag 1740
gatgacagac aggtggaact gccagtgtag agggaattct aagctggacg cacagaacag 1800
tctcttcgtg ggaggagaca ttatggggcg tccaccacca cccctccctg gccatcctcc 1860
tggaatgtgg tctgccctcc accagagctc ctgcctgcca ggactggacc agagcagcca 1920
ggctggggcc cctctgtctc aacccgcaga cccttgactg aatgagagag gccagaggat 1980

gctccccatg ctgccactat ttattgtgag ccctggaggc tcccatgtgc ttgaggaagg 2040
ctggtgagcc cggctcagga ccctcttccc tcaggggctg cagcctcctc tcactccctt 2100
ccatgccgga acccaggcca gggacccacc ggcctgtggt ttgtgggaaa gcagggtgca 2160
cgctgaggag tgaaacaacc ctgcacccag agggcctgcc tggtgccaag gtatcccagc 2220
ctggacaggc atggacctgt ctccagacag aggagcctga agttcgtggg gcgggacagc 2280
ctcggcctga tttcccgtaa aggtgtgcag cctgagagac gggaagagga ggcctctgca 2340
cctgctggtc tgcactgaca gcctgaaggg tctacaccct cggctcacct aagtccctgt 2400
gctggttgcc aggcccagag gggaggccag ccctgccctc aggacctgcc tgacctgcca 2460
gtgatgccaa gagggggatc aagcactggc ctctgcccct cctccttcca gcacctgcca 2520
gagcttctcc agcaggccaa gcagaggctc ccctcatgaa ggaagccatt gcactgtgaa 2580
cactgtacct gcctgctgaa cagcctcccc ccgtccatcc atgagccagc atccgtccgt 2640
cctccactct ccagcctctc cccagcctcc tgcactgagc tggcctcacc agtcgactga 2700
gggagcccct cagccctgac cttctcctga cctggccttt gactccccgg agtggagtgg 2760
ggtgggagaa cctcctgggc cgccagccag agccgctctt taggctgtgt tcttcgccca 2820
ggtttctgca tcttccactt tgacattccc aagagggaag ggactagtgg gagagagcaa 2880
gggaggggag ggcacagaca gagagcctac agggcgagct ctgactgaag atgggccttt 2940
gaaatatagg tatgcacctg aggttggggg agggtctgca ctcccaaacc ccagcgcagt 3000
gtcctttccc tgctgccgac aggaacctgg ggctgagcag gttatccctg tcaggagccc 3060
tggactgggc tgcatctcag ccccacctgc atggtatcca gctcccatcc acttctcacc 3120
cttctttcct cctgaccttg gtcagcagtg atgacctcca actctcaccc accccctcta 3180
ccatcacctc taaccaggca agccagggtg ggagagcaat caggagagcc aggcctcagc 3240
ttccaatgcc tggagggcct ccactttgtg gccagcctgt ggtgctggct ctgaggccta 3300
ggcaacgagc gacagggctg ccagttgccc ctgggttcct ttgtgctgct gtgtgcctcc 3360
tctcctgccg ccctttgtcc tccgctaaga gaccctgccc tacctggccg ctgggccccg 3420
tgactttccc ttcctgccca ggaaagtgag ggtcggctgg ccccaccttc cctgtcctga 3480
tgccgacagc ttagggaagg gcactgaact tgcatatggg gcttagcctt ctagtcacag 3540
cctctatatt tgatgctaga aaacacatat ttttaaatgg aagaaaaata aaaaggcatt 3600
cccccttcat ccccctacct taaacatata atattttaaa ggtcaaaaaa gcaatccaac 3660
ccactgcaga agctcttttt gagcacttgg tggcatcaga gcaggaggag ccccagagcc 3720
acctctggtg tcccccaggc tacctgctca ggaacccctt ctgttctctg agaactcaac 3780
agaggacatt ggctcacgca ctgtgagatt ttgtttttat acttgcaact ggtgaattat 3840
tttttataaa gtcatttaaa tatctattta aaagatagga agctgcttat atatttaata 3900
ataaaagaag tgcacaagct gccgttgacg tagctcgag 3939

<210> 2
<211> 18
<212> ARN
<213> Séquence artificielle

<220>
<223> Description de la séquence
      artificielle:Oligonucléotide de synthèse

<400> 2
gggagggugc uguaccga 18

<210> 3
<211> 16
<212> ARN
<213> Séquence artificielle

<220>
<223> Description de la séquence
      artificielle:Oligonucléotide de synthèse

<400> 3
ucuggagcag guuaau 16

<210> 4
<211> 18
<212> ARN
<213> Séquence artificielle

<220>
<223> Description de la séquence
      artificielle:Oligonucléotide de synthèse

<400> 4
acacgaugga auucuucc                                                    18


<210> 5
<211> 18
<212> ARN
<213> Séquence artificielle

<220>
<223> Description de la séquence
      artificielle:Oligonucléotide de synthèse

<400> 5
uuguggcggu uaggguag                                                    18

## Description

La présente invention concerne des produits de combinaison comprenant (i) au moins une substance capable d'inhiber l'activité de CSF-1 et/ou au moins un acide nucléique, comprenant au moins une séquence codant pour une substance capable d'inhiber l'activité de CSF-1 et (ii) au moins une substance ayant une activité cytotoxique et/ou au moins un acide nucléique, comprenant au moins une séquence codant pour une substance ayant une activité cytotoxique. La présente invention concerne également des oligonucléotides capables d'inhiber l'expression de CSF-1. La présente invention est particulièrement utile dans le cadre de la mise en oeuvre d'un traitement antitumoral.

A ce jour, les résultats les plus encourageants obtenus dans le cadre de traitements antitumoraux concernent des traitements combinés qui associent un traitement à base de composés chimiques (chimiothérapie) et un traitement reposant sur l'utilisation de rayonnements (radiothérapie). Outre les désagréments importants qu'occasionne chez le patient ce type de traitement, on constate de 10 à 50 % de rechutes (suivant la précocité du traitement).

Par ailleurs, des travaux conduits dans le domaine du cancer ont proposé d'adapter les protocoles de thérapie génique à la thérapie antitumorale. A cet égard, on peut par exemple citer les travaux de Meneguzzi et al. (1991, Virology, 181, 61-69) relatifs à l'immunisation contre des cellules tumorales à l'aide d'un vecteur recombinant de la vaccine exprimant les gènes E6 et E7 du virus du papillome humain de type 16 (voir également les brevets US 5,744,133 ou US 6,007,806) ou de Leroy et al. (1998, Res.Immunol. 149, : 681-684) qui ont montré que la production de cytokines sur les sites de la tumeur après administration intra-tumorale de vecteurs viraux recombinants permet l'induction d'une réponse immunitaire associée à une inhibition de la croissance tumorale. On peut également citer les résultats obtenus à l'aide de cellules génétiquement modifiées capables d'exprimer l'IL2 (Roschlitz et al., 1999, Cancer Gene Ther., 6, 271-281) ou de vecteurs viraux exprimant un antigène tumoral et une interleukine (Bizouarne et al., 1996, In « Breast cancer. Advances in Biology and Therapeutics », F. Calvo, M. Crepin and H. Magdalenat eds., 303-308 - pour une revue, voir Zhang ,1999, Cancer Gene Therapy, 6, 113-138).

Toutefois, la réponse antitumorale observée dans le cadre de ces différents traitements, bien qu'encourageante, n'est pas meilleure que les réponses cliniques observées dans le cadre de traitements classiques (i.e. chimiothérapie, radiothérapie).

Il est par conséquent souhaitable de disposer de nouveaux produits et/ou de nouvelles méthodes permettant la mise en oeuvre de traitements antitumoraux efficaces, aisés à mettre en place, permettant un contrôle prolongé du volume tumoral et l'augmentation du taux de survie des patients traités et présentant peu ou pas d'effets secondaires autres que l'activité cytotoxique recherchée.

Le CSF-1 (Colony stimulating factor 1) est une cytokine notamment exprimée par les monocytes, c'est un facteur de différenciation, de croissance et de survie de ces cellules. Son récepteur est le produit du proto-oncogène c-fms (Sherr et al., 1990, Blood, 75, 1-12). Le CSF-1 est également exprimé de façon physiologique dans l'endomètre et le placenta humain ainsi que par certaines cellules tumorales. Des travaux réalisés par immunohistochimie et hybridation in situ ont mis en évidence une spécificité d'expression du CSF-1 dans les cellules tumorales invasives alors qu'une telle production n'est pas observée dans les cellules tumorales intra-canalaires, ou non-invasives (Scholl et al. J. Natl. Cancer. Inst., 1994, 86,120-126: J. Cellular. Biochem., 1992, 50, 350-356; Mol. Carcinog., 1993, 7207-211). La production de CSF-1 par les cellules tumorales invasives se traduit notamment par une augmentation de sa concentration dans le plasma des patients, qui peut alors atteindre plus de 1000 pg/ml contre moins de 300 pg/ml chez les sujets normaux. Une telle concentration témoigne d'un stade avancé de la maladie et d'un pronostic à court terme défavorable (Scholl et al, Br. J. Cancer. 1994, 69, 342-346; Scholl et al, Breast Cancer Res. Treat., 1996 39:275-283).

Par ailleurs, il a été montré que le CSF-1 stimule la mobilité et l'invasivité des cellules tumorales (Dorsch et al, Eur. J. Imm., 1993; 23,186-90; Wang et al J. Immunol., 1988, 141, 575-579; Filderman et al, Cancer Research, 1992, 52, 3661-3666). Le CSF-1 a également un effet chimiotactique, sur les précurseurs de la lignée myéloïde, qui favorise l'infiltration des monocytes dans la tumeur. Pourtant, la présence de ces monocytes ne suffit pas pour observer une destruction de la tumeur par le système immunitaire (Dorsch et al., 1993, Eur. J. Immunol, 23, 186-190). Il semble en effet que le CSF-1, aux taux sériques élevés communément retrouvés chez des patients porteurs de tumeurs solides du sein, des ovaires ou du pancréas, oriente la différenciation de ces monocytes en macrophages et non pas en cellules dendritiques capables de présenter les antigènes tumoraux et ainsi d'amorcer une réponse immunitaire cytotoxique efficace dirigée contre les cellules tumorales (Scholl et al., 1996, Breast Cancer. Res. Treat., 39, 275-283; Baron et al, J. Cell. Sci., 2000, 114, 999-1010).

La présente demande concerne des produits de combinaison permettant d'induire la mort des cellules tumorales et de contrôler la concentration de CSF-1 et/ou l'activité du CSF-1 chez les patients traités. L'association de ces deux propriétés est particulièrement intéressante dans le cadre d'un traitement antitumoral. En effet, les déposants ont mis en évidence que les patients présentant les meilleurs réponses cliniques à un traitement antitumoral, consistant à les immuniser contre les cellules tumorales à l'aide d'un vecteur codant pour la mucine MUC-1, étaient ceux dont le taux sérique de CSF-1 était le plus faible. Plus particulièrement, de tels produits de combinaison permettent d'inhiber ou de retarder la prolifération cellulaire en induisant la mort spécifique des cellules, notamment tumorales, une meilleure présentation des antigènes et/ou une meilleure stimulation des cellules immunes de l'organisme hôte. La présente invention offre une alternative avantageuse et efficace aux techniques de l'art antérieur, notamment pour traiter le cancer de l'homme ou de l'animal.

Ainsi, la présente invention concerne un produit de combinaison comprenant :
(i) au moins une substance capable d'inhiber l'activité de CSF-1 et/ou au moins un acide nucléique, comprenant au moins une séquence codant pour une substance capable d'inhiber l'activité de CSF-1 et,
(ii) au moins une substance ayant au moins une activité cytotoxique et/ou au moins un acide nucléique, comprenant au moins une séquence codant pour une substance ayant au moins une activité cytotoxique.

Par "substance capable d'inhiber l'activité de CSF-1" on entend désigner les substances capables d'inhiber l'expression de CSF-1 et les substances capables d'inhiber la liaison de CSF-1 à son récepteur.

Par "capable d'inhiber l'expression de CSF-1" on entend signifier que l'action de ladite substance entraîne une diminution de la quantité de CSF-1 produite par la cellule cible. La capacité d'une substance à inhiber l'expression de CSF-1 peut notamment être vérifiée *in vitro* en mettant en contact des cellules exprimant CSF-1 avec une substance capable d'inhiber l'expression de CSF-1, puis en mesurant la quantité de CSF-1 exprimée par ladite cellule (Birchenall-Roberts et al., J. Immunol. 1990, 145, 3290-3296).

Selon un mode de réalisation préféré de l'invention la substance capable d'inhiber l'activité de CSF-1 est un oligonucléotide.

Parmi les oligonucléotides capables d'inhiber l'expression de CSF-1, on peut notamment citer l'oligonucléotide décrit par Birchenall-Roberts et al. (J. Immunol., 1990, 145, 3290-3296). Cet oligonucléotide capable de s'hybrider au niveau du codon d'initiation de la séquence codant CSF-1 (i.e. la région comprise entre le nucléotide en position 97 et le nucléotide en position 99 inclus de SEQ ID NO:1) permet d'inhiber efficacement l'expression de CSF-1 par les cellules cibles.

Les déposants ont également identifiés de nouveaux oligonucléotides, capables d'inhiber l'expression de CSF-1.

Ainsi, la présente invention concerne également un oligonucléotide, capable d'inhiber l'expression de CSF-1, de 8 à 100 nucléotides de long caractérisé en ce qu'il est capable de s'hybrider à la région comprise entre le nucléotide en position 121 et le nucléotide en position 450 inclus, préférentiellement entre le nucléotide en position 131 et le nucléotide en position 391 inclus, et de manière tout à fait préférée entre le nucléotide en position 135 et le nucléotide en position 152 inclus ou entre le nucléotide en position 284 et le nucléotide en position 301 inclus ou entre le nucléotide en position 341 et le nucléotide en position 358 inclus de SEQ ID NO:1.

Le terme oligonucléotide fait référence à un polymère de nucléotides simple brin. Les nucléotides sont bien connus de l'homme de l'art, ils sont composés d'au moins une base nucléique, d'au moins un groupement ose et d'au moins un groupement phosphate.

Les oligonucléotides selon l'invention peuvent être modifiés, notamment pour améliorer leur stabilité *in vivo*, leur liaison à la séquence cible et/ou leur capture par les cellules.

Parmi les oligonucléotides modifiés utilisables dans le cadre de la présente invention, on peut notamment citer les oligonucléotides comprenant une ou plusieurs liaisons phosphorothioates, phosphotriesters et/ou methylphosphonates. Selon un mode de réalisation préféré, l'oligonucléotide selon l'invention comprendra une ou plusieurs liaisons phosphorothioates.

Parmi les oligonucléotides modifiés, on peut également citer ceux comprenant un ou plusieurs dérivés de l'adénine, de la guanine, de la cytosine, de la thymine et ou de l'uracile. Ces dérivés comprennent mais ne se limitent pas à l'hypoxanthine, la 5-(1-propynyl)-2'-deoxycytidine, la 5-(1-propynyl)-2'deoxyuridine, la 6-méthyladénine, la 5-méthylcytosine, le 5-bromouracile, le 5-Hydroxyméthyluracile, la 8-azaguanine, la 7-déazaguanine et la 2,6-diaminopurine. Selon un mode de réalisation préféré de l'invention, l'oligonucléotide selon l'invention comprend une ou plusieurs 5-(1-propynyl)-2'deoxyuridine et/ou une ou plusieurs 5-(1-propynyl)-2'-deoxycytidine.

Parmi les oligonucléotides modifiés on peut également citer les oligonucléotides dont un ou plusieurs groupements oses sont substitués. Par "groupement ose substitué", on entend désigner les groupements oses dont l'hydrogène ou l'hydroxyle en position 2', 3' et/ou 5' est remplacé par une autre fonction chimique. Selon un mode de réalisation préféré de l'invention, l'oligonucléotide selon l'invention comprend un ou plusieurs radicaux 2'-methoxyethoxyl, 2'-propoxy et/ou 2'-fluoro.

D'autres oligonucléotides modifiés sont connus de l'homme de l'art et sont utilisables dans le cadre de la présente invention. On peut notamment citer les oligonucléotides décrits dans le brevet US 6,238,921 dont le contenu est incorporé ici par référence.

Les oligonucléotides peuvent être préparés selon des techniques bien connues de l'homme de l'art, notamment par les techniques de synthèse en phase solide (pour une revue voir Sproat et al., 1984, Solid-phase synthesis of oligodeoxyribonucleotides by the phosphotriester method. In Oligonucleotide Synthesis - A practical approach, Gait,M.J. Ed.., IRL Press, Oxford pp.83-115).

Par "capable de s'hybrider", on entend signifier que l'oligonucléotide selon l'invention est capable de se lier spécifiquement à une séquence cible. L'hybridation est la conséquence de liaisons hydrogènes entre les nucléotides de l'oligonucléotide d'une part et les nucléotides de la séquence cible d'autre part. Pour que la liaison entre deux nucléotides soit possible, il faut que ces deux nucléotides soient complémentaires. Il est connu de l'homme du métier qu'il n'est pas nécessaire que 100% des nucléotides composant un oligonucléotide soient complémentaires aux nucléotides de la séquence cible pour qu'il y est hybridation. Selon un mode de réalisation de l'invention, l'oligonucléotide selon l'invention aura plus de 80%, préférentiellement plus de 90% et de manière tout à fait préféré 100% de ses nucléotides complémentaires aux nucléotides de la séquence cible.

L'hybridation entre un oligonucléotide et une séquence cible peut être vérifiée selon des méthodes bien connues de l'homme du métier. On peut notamment citer les méthodes décrites par Southern et al. (Ciba Found.Symp., 1997, 209, 38-44).

La détermination de la séquence d'un oligonucléotide capable de s'hybrider à une séquence cible donnée est à la portée de l'homme du métier qui peut éventuellement s'aider d'un des logiciels conçus à cet effet (Song et al., Acta Pharmacol Sin., 2000, 21, 80-86).

Par "séquence cible", on entend désigner une séquence d'ADN génomique codant pour un polypeptide dont on souhaite moduler l'expression, la ou les séquences d'ADN génomique impliquées dans la régulation de la transcription de ladite séquence codant pour un polypeptide dont on souhaite moduler l'expression, ainsi que le ou les ARNm transcrits de ces séquences d'ADN génomique.

Avantageusement, l'oligonucléotide selon l'invention comprend de 8 à 30 nucléotides, de préférence de 12 à 25 nucléotides et de manière tout à fait préférée de 16 à 19 nucléotides.

Dans le cas particulier ou l'oligonucléotide selon l'invention est composé uniquement d'acides desoxyribonucléiques il pourra être transcrit à partir d'une séquence d'acide nucléique. Ainsi l'invention concerne également un acide nucléique comprenant une séquence codant pour un oligonucléotide selon l'invention.

Parmi les substances capables d'inhiber la liaison de CSF-1 à son récepteur, on peut citer par exemple les anticorps, peptides, lipides ou sucres capables de se lier à CSF-1 ou à son récepteur.

Selon un mode de réalisation préféré, la substance capable d'inhiber la liaison de CSF-1 à son récepteur est un anticorps. Par "anticorps" on entend désigner les anticorps polyclonaux, les anticorps monoclonaux, les anticorps chimériques, les scFv et les fragments d'anticorps. Plus particulièrement, par «fragment» d'anticorps on entend désigner les fragments F(ab)2, Fab', Fab, Fv, sFv (Blazar et al., 1997, J. of Immunology 159, 5821-5833 ; Bird et al., 1988, Science 242, 423-426) et dAbs (Ward et al., 1989, Nature 341, 544) d'un anticorps natif qu'il soit d'origine polyclonale ou monoclonale (voir par exemple Monoclonal Antibodies : A manual of Techniques and Applications H. Zola (CRC Press, 1988) et Monoclonal Hybridoma Antibodies : Techniques and Applications, J. Hurrell (CRC Press, 1982)). Par ailleurs, une revue générale des techniques concernant la synthèse de fragments d'anticorps qui retiennent la spécificité de liaison de l'anticorps natif est accessible dans Winter et al. (1991, Nature 349, 293-299). Certains anticorps capables de se lier à CSF-1 ou à son récepteur sont disponibles commercialement (par exemple auprès de Oncogene Science, Manhasset, NY, USA). On peut également citer l'anticorps monoclonal décrit par Birchenall-Roberts et al. (J. Immunol., 1990, 145, 3290-3296).

Selon un autre mode de réalisation de l'invention, , la substance capable d'inhiber la liaison de CSF-1 à son récepteur est un peptide capable de se lier à CSF-1 ou au récepteur de CSF-1. Ces peptides peuvent être préparés selon des procédures disponibles dans l'art antérieur. Par exemple, ces peptides peuvent être préparés par des techniques de Phage display (Smith, G. P., Curr. Opin. Biotechnol. 2: 668-673 (1991).

Par "substance ayant au moins une activité cytotoxique », on entend indiquer que la substance considérée est capable d'induire ou d'activer une réponse immune dirigée spécifiquement contre une cellule cible ou d'inhiber la croissance et / ou la division d'une telle cellule. Selon un cas préféré, cette activité cytotoxique se traduit par la mort de ladite cellule. Dans un mode de réalisation préféré de l'invention, ladite cellule cible est une cellule tumorale (l'activité cytotoxique est alors appelée activité antitumorale).

L'activité cytotoxique d'une substance donnée, notamment une activité antitumorale, peut être évaluée *in vitro* par la mesure de la survie cellulaire soit par des tests de viabilité à court terme (tel que par exemple le test au bleu tryptan ou MTT), soit par des tests de survie clonogénique (formation de colonies) (Brown et Wouters, 1999, Cancer Research, 59, 1391-1399) ou *in vivo* par la mesure de la croissance des tumeurs (taille et/ou volume) dans un modèle animal (Ovejera et Houchens, 1981, Semin. Oncol., 8, 386-393).

Selon un mode de réalisation de l'invention la substance ayant au moins une activité cytotoxique est choisie parmi les substances interagissant avec l'ADN, les antimétabolites, les inhibiteurs de topo-isomérases et les agents du fuseau.

Les substances interagissant avec l'ADN sont des substances couramment utilisées dans le cadre des traitements anticancéreux. On peut notamment citer les alkylants, les donneurs de méthyl (par exemple les nitroso-urées), les organoplatines (notamment le cisplatine, le carboplatine et l'oxaliplatine) et les agents scindants l'ADN.

Les antimétabolites agissent en inhibant le métabolisme cellulaire et sont également bien connus de l'homme du métier. On peut citer notamment les antifoliques comme par exemple le méthotrexate et le raltitrexed, les antipuriques comme la cladribine, la fludarabine, la mercaptopurine et la pentostatine, et les antipyrimidiques comme par exemple la cytarabine, le Fluorouracil, et la Gemcitabine.

Parmi les inhibiteurs de topo-isomérases, on peut citer notamment, les inhibiteurs de topo-isomérases I (e.g. l'irinotécan et le topotécan) et les inhibiteurs des topo-isomérases II (e.g. les anthracyclines).

Les agents du fuseau sont des substances qui bloquent la formation ou la disparition du fuseau mitotique entraînant ainsi une inhibition de la mitose cellulaire. Parmi ceux-ci, on peut citer la vincristine, la vinblastine et les taxoïdes.

Selon un mode de réalisation préférée de l'invention la substance ayant au moins une activité cytotoxique est choisie parmi les antigènes associés aux tumeurs (AAT). Certaines cellules tumorales expriment des antigènes spécifiques. Ces AAT peuvent être utilisés dans le cadre d'un traitement immunothérapeutique, pour initier une réponse immunitaire contre les cellules tumorales (pour une revue, voir par exemple, Bacchia et al. Haematologica, 2000, 85, 1172-206).

Les AAT utilisables dans le cadre de l'invention comprennent mais ne se limitent pas à MART-1 (Kawakami et al. J. Exp. Med. 180:347-352, 1994), MAGE-1, MAGE-3, GP-100, (Kawakami et al. Proc. Nat. Acad. Sci. U.S.A. 91:6458-6462, 1994), CEA, la tyrosinase (Brichard et al. J. Exp. Med. 178:489, 1993),MUC-1, MUC-2, l'oncogène ras mutée, l'oncogène p53, CA-125, PSA, PSMA, PAP et c-erb/B2 (Boon et al., Ann. Rev. Immunol. 12:337, 1994).

Selon un autre mode de réalisation de l'invention, la substance ayant au moins une activité cytotoxique est choisi parmi les cytokines, les polypeptides présentant une activité de chimio-attraction (i.e. chimiokines), les protéines codées par un gène appelé « gène suicide », les facteurs protéiques anti-angiogéniques et les polypeptides présentant une activité d'activation de l'apoptose cellulaire.

Les cytokines sont des molécules naturellement produites à la suite d'une stimulation antigénique ou d'une réaction inflammatoire (Gillis and Williams, 1998, Curr. Opin. Immunol., 10, 501-503) dont l'utilité dans le cadre du traitement de certains cancers a été montrée notamment par Oettger (Curr. Opin. Immunol., 1991, 3, 699-705). Selon cette première variante de l'invention, la substance ayant au moins une activité cytotoxique désignera préférentiellement une cytokine choisie parmi les interférons α, β et γ, les interleukines, et notamment l'IL-2, l'IL-4, l'IL-6, l'IL-10 ou l'IL-12, les facteurs nécrosant des tumeurs (TNF) et les facteurs stimulateurs de colonies (par exemple GM-CSF, C-CSF, M-CSF).

Selon un mode de réalisation préféré, ladite cytokine est sélectionnée parmi l'interleukine-2 (IL-2) et l'interféron gamma (IFN-γ). L'interleukine-2 est notamment responsable de la prolifération des lymphocytes T activés, de la multiplication et de l'activation des cellules du système immunitaire (pour la séquence en acide nucléique voir notamment EP0206939). L'IFN-γ active les cellules phagocytaires et accroît l'expression des antigènes de surface de classe I et II du complexe majeur d'histocompatibilité (pour la séquence en acide nucléique voir notamment EP0206920). Ces séquences en acides nucléiques sont incorporées à la demande par référence.

Selon un mode de réalisation particulier, le produit de combinaison selon l'invention est caractérisé en ce qu'il comprend au moins deux substances ayant au moins une activité cytotoxique : l'interleukine-2 (IL-2) et l'interféron gamma (IFN-γ).

Selon une autre variante de l'invention, la substance ayant au moins une activité cytotoxique est un polypeptide présentant une activité de chimio-attraction (i.e. chimiokines). Les chimiokines constituent une sous classe de la famille des cytokines. Elles se distinguent des autres cytokines par leur propriété chimio-attractive, notamment lors des processus naturels de chimiotactisme, et notamment d'attraction des cellules du système immunitaire vers les tissus dans lesquels siège l'inflammation ou l'infection, ainsi que par leurs propriétés anti-angiogéniques.

Les chimiokines sont des protéines de faible poids moléculaire (entre 8 et 10 kd ), de petite taille (de 70 à 80 acides aminés) dont les séquences en acides aminés présentent un faible taux d'homologie (variant de 10 à 70 % selon les chimiokines considérées) permettant de définir à ce jour environ 50 chimiokines différentes. Ces chimiokines peuvent néanmoins être subdivisées en 4 grandes familles relatives à la position des résidus cystéines qu'elles renferment. Les familles α dont l'extrémité N-terminale comprend 2 cystéines séparées par un acide aminé unique (chimiokines de type IL-8, NAP-2, GCP-2) et ∃ dont l'extrémité N-terminale comprend 2 cystéines adjacentes (chimiokines de type RANTES, MIP1, MCP1) sont les mieux caractérisées (Horuk, R., 1994, Trends Pharmacol. Sci., 15, pages 159-165 ; Murphy, P.M., 1994, Annu. Rev. Immunol., 12, pages 593-633).

Dans le cadre de la présente invention, la chimiokine préférée est la chimiokine de type MIP 1, et plus particulièrement sélectionnée parmi le groupe consistant en les chimiokines MIP1α et MIP1β dont les propriétés ont été mises en évidence par Wolpe et al (1988, J. Exp. Med, 167, 570-581).

MIP1α , dont les séquences en acides nucléiques et peptidique sont décrites dans Obaru et al. (1986, J. Biochem., 99, 885-894), dont le contenu est incorporé par référence dans la présente demande, est produite par les lymphocytes T et les monocytes. Elle permet la chimio-attraction des éosinophiles et des lymphocytes T au cours des infections des voies respiratoires ; des monocytes et des neutrophiles au cours d'arthrites rhumatoïdales, d'inflammations du système digestif ou de méningites d'origine bactérienne. En outre, elle inhibe la prolifération des précurseurs hématopoïétiques.

MIP1β, dont les séquences en acides nucléiques et peptidique sont décrites dans Brown et al. (1989, J. Immunol., 142, 679-68), dont le contenu est incorporé par référence dans la présente demande, est également produite par les lymphocytes T et les monocytes. Elle exerce ses propriétés chimio-attractives sur les monocytes et les neutrophiles dans les cas arthrites osseuses et les méningites bactériennes. Comme MIP1α, elle inhibe la prolifération des précurseurs hématopoïétiques.

Il existe des variants naturels desdites protéines MIP1α et MIP1β qui sont connus de l'homme du métier et qui portent par exemple les noms GOS19, LD78, pAT464 , TY5 (de souris) ou SIS α (de souris) pour MIP1α ou pAT744, Act-2, G-26, H-400 (de souris) ou hSIS γ (de souris) pour MIP1β. Dans le cas particulier de MIP1β, on choisira par exemple la séquence correspondant à Act-2 (Lipes et al., 1988, PNAS, 85, 9704-9708), dont le contenu est incorporé ici en référence).

Selon une autre variante de l'invention, la substance ayant au moins une activité cytotoxique est un polypeptide codé par un gène appelé « gène suicide ». Plusieurs études ont permis d'identifier des polypeptides qui ne sont pas toxiques en tant que tels mais qui présentent des propriétés enzymatiques catalytiques capables de transformer une substance inactive (pré-drogue) , par exemple un nucléoside ou un analogue de nucléoside, en substance hautement toxique pour la cellule, par exemple un nucléoside modifié qui peut être incorporé dans les chaînes d'ADN ou d'ARN en élongation, avec pour conséquence, notamment, l'inhibition de la division cellulaire ou des dysfonctionnements cellulaires conduisant à la mort de la cellule renfermant de tels polypeptides. Les gènes codant pour de tels polypeptides sont dits « gènes suicides ». De nombreux couples gène suicide/pré-drogue sont actuellement disponibles. On peut citer plus particulièrement, les couples :
- la thymidine kinase du virus herpès simplex de type 1 (TK HSV-1) et l' acyclovir ou le ganciclovir (GCV) (Caruso et al., 1993, Proc. Natl. Acad. Sci. USA 90, 7024-7028 ; Culver et al., 1992, Science 256, 1550-1552 ; Ram et al., 1997, Nat. Med. 3, 1354-1361);
- le cytochrome p450 de rat et la cyclophosphophamide (Wei et al., 1994, Human Gene Therapy 5, 969-978) ;
- la purine nucleoside phosphorylase d'Escherichia coli (E. Coli) et la 6-methylpurine deoxyribonucleoside (Sorscher et al., 1994, Gene Therapy 1, 233-238) ;
- la guanine phosphoribosyl transférase d'E. coli et la 6- thioxanthine (Mzoz et Moolten, 1993, Human Gene Therapy 4, 589-595) et
- la cytosine désaminase (CDase) et la 5-fluorocytosine (5FC).

Selon un cas avantageux, l'invention concerne le cas selon lequel ladite substance ayant au moins une activité cytotoxique présente au moins une activité enzymatique sélectionnée parmi l'activité thymidine kinase, l'activité purine nucléoside phosphorylase, l'activité guanine ou uracile ou orotate phosphoribosyl transférase et l'activité cytosine désaminase.

Plus particulièrement, la CDase est un enzyme qui intervient dans la voie métabolique des pyrimidines par laquelle la cytosine exogène est transformée par le biais d'une désamination hydrolytique en uracile. Des activités CDases ont été mises en évidence chez les procaryotes et les eucaryotes inférieurs (Jund et Lacroute, 1970, J. Bacteriol. 102, 607-615 ; Beck et al., 1972, J. Bacteriol. 110, 219-228 ; De Haan et al., 1972, Antonie van Leeuwenhoek 38, 257-263 ; Hoeprich et al., 1974, J. Inf. Dis. 130, 112-118 ; Esders et Lynn, 1985, J. Biol. Chem. 260, 3915-3922) mais elles sont absentes chez les mammifères (Koechlin et al., 1966, Biochem Pharmacol. 15, 435-446 ; Polak et al., 1976, Chemotherapy 22, 137-153). Les gènes FCY1 de Saccharomyces cerevisiae (S. cerevisiae) et codA d'E. coli codant respectivement pour la CDase de ces deux organismes sont connus et leurs séquences publiées (EP 402 108 ; Erbs et al., 1997, Curr. Genet. 31, 1-6 ; WO93/01281). La CDase désamine également un analogue de la cytosine, la 5-fluorocytosine (5-FC) en 5-fluorouracile (5-FU) qui est un composé hautement cytotoxique notamment lorsqu' il est converti en 5-fluoro-UMP (5-FUMP). Les cellules dépourvues d'activité CDase, en raison soit d'une mutation inactivante du gène codant pour l'enzyme, soit de leur déficience naturelle pour cette enzyme (par exemple les cellules mammifères) sont résistantes au 5-FC (Jund et Lacroute, 1970, J. Bacteriol. 102, 607-615 ; Kilstrup et al., 1989, J. Bacteriol., 171, 2124-2127). Par contre, il a été montré qu'il est possible de transmettre la sensibilité au 5-FC à des cellules mammifères dans lesquelles la séquence codant pour une activité CDase a été transférée (Huber et al., 1993, Cancer Res. 53, 4619-4626 ; Mullen et al., 1992, Proc. Natl. Acad. Sci. USA 89, 33-37 ; WO 93/01281). De plus, dans ce cas, les cellules avoisinantes non transformées deviennent également sensibles au 5-FC (Huber et al., 1994, Proc. Natl. Acad. Sci. USA 91, 8302-8306). Ce phénomène, appelé effet de voisinage (bystander en anglais), est dû à l'excrétion par les cellules exprimant l'activité CDase, de 5-FU qui intoxique les cellules voisines par simple diffusion à travers la membrane cellulaire. Cette propriété de diffusion passive du 5-FU constitue un avantage par rapport au système de référence tk/GCV pour lequel l'effet de voisinage nécessite un contact avec les cellules qui expriment tk (Mesnil et al., 1996, Proc. Natl. Acad. Sci. USA 93, 1831-1835). Cet effet constitue par conséquent un atout supplémentaire de l'utilisation de la CDase dans le cadre de la thérapie génique, notamment anticancéreuse.

Cependant, la sensibilité au 5-FC varie beaucoup selon les lignées cellulaires. Une faible sensibilité est observée par exemple dans des lignées tumorales humaines PANC-1 (carcinome de pancréas) et SK-BR-3 (adénocarcinome du sein) transduites par un rétrovirus exprimant le gène codA d'E. Coli (Harris et al., 1994, Gene Therapy 1, 170-175). Ce phénomène indésirable pourrait s'expliquer par l'absence ou la faible conversion endogène du 5-FU formé par l'action enzymatique de la CDase en 5-FUMP cytotoxique. Cette étape, normalement assurée dans les cellules mammifères par l'orotate phospho-rybosyl transférase (Peters et al., 1991, Cancer 68, 1903-1909), peut être absente dans certaines tumeurs et rendre ainsi la thérapie génique, basée sur la CDase, inopérante. Chez les procaryotes et eucaryotes inférieurs, l'uracile est transformée en UMP par l'action de l'uracile phosphoribosyl transférase (présentant par conséquent une activité UPRTase). Cette enzyme convertit également le 5-FU en 5-FUMP. Ainsi des mutants fur1 de la levure S. cerevisiae sont résistants à de fortes concentrations de 5-FU (10 mM) et de 5-FC (10 mM) car en absence d'activité UPRTase, le 5-FU, provenant de la désamination du 5-FC par la CDase, n'est pas transformé en 5-FUMP cytotoxique (Jund et Lacroute, 1970, J. Bacteriol. 102, 607-615). Les gènes upp et FUR1 codant pour l'UPRTase respectivement d'E. coli et de S. cerevisiae ont été clonés et séquencés (Andersen et al., 1992, Eur. J. Biochem. 204, 51-56 ; Kern et al., 1990, Gene 88, 149-157).

Selon un mode de réalisation de la présente invention, la substance ayant au moins une activité cytotoxique a une activité UPRTase ce qui signifie que ledit polypeptide est capable de convertir l'uracile ou un de ses dérivés en un analogue monophosphaté et, en particulier la 5-FU en 5-FUMP.

L'UPRTase dont il est question dans la présente invention peut être d'une origine quelconque, notamment procaryotique, fongique ou de levure. A titre illustratif, les séquences d'acide nucléique codant pour les UPRTases d'E. coli (Anderson et al., 1992, Eur. J. Biochem 204, 51-56), de Lactococcus lactis (Martinussen et Hammer, 1994, J. Bacteriol. 176, 6457-6463), de Mycobacterium bovis (Kim et al., 1997, Biochem Mol. Biol. Int 41, 1117-1124) et de Bacillus subtilis (Martinussen et al., 1995, J. Bacteriol. 177, 271-274) peuvent être utilisées dans le cadre de l'invention. Mais on préfère tout particulièrement mettre en oeuvre une UPRTase de levure et notamment celle codée par le gène FUR1 de S. cerevisiae dont la séquence divulguée dans Kern et al. (1990, Gene 88, 149-157) est introduite ici par référence. A titre indicatif, les séquences des gènes et celles des UPRTases correspondantes peuvent être trouvées dans la littérature et les banques de données spécialisées (SWISSPROT, EMBL, Genbank, Medline...).

Par ailleurs, la demande WO9954481 décrit un gène FUR1 dépourvu de 105 nucléotides en 5' de la partie codante permettant la synthèse d'une UPRTase délétée des 35 premiers résidus en position N-terminale et débutant à la méthionine en position 36 dans la protéine native. Le produit d'expression du gène mutant, désigné FUR1)105, est capable de complémenter un mutant fur1 de S. cerevisiae. En outre, le mutant tronqué présente une activité UPRTase supérieure à celle de l'enzyme native. Ainsi, selon un mode de réalisation particulièrement avantageux, le polypeptide codé selon l'invention est un mutant de délétion d'une UPRTase native. La délétion est de préférence localisée dans la région N-terminale de l'UPRTase d'origine. Elle peut être totale (concerner l'ensemble des résidus de ladite région N-terminale) ou partielle (concerner un ou plusieurs résidus continus ou non dans la structure primaire). D'une manière générale, un polypeptide est constitué de parties N-terminale, centrale et C-terminale, chacune représentant environ le tiers de la molécule. Par exemple, l'UPRTase de S. cerevisiae ayant 251 acides aminés, sa partie N-terminale est constituée des 83 premiers résidus débutant à la méthionine dite initiatrice située en première position de la forme native. Quant à l'UPRTase d'E. coli, sa partie N-terminale couvre les positions 1 à 69.

En outre, les demandes de brevet WO96/16183 et WO9954481 décrivent l'utilisation d'une protéine de fusion codant pour une enzyme à deux domaines ayant les activités CDase et UPRTase et démontrent que le transfert d'un gène hybride codA::upp ou FCY1::FUR1 ou FCY1::FUR1Δ105 porté par un plasmide d'expression augmente la sensibilité au 5-FC de cellules B16 transfectées. Les séquences protéiques et nucléiques décrites dans ces deux demandes sont incorporées dans la description de la présente demande. Selon ce mode de réalisation, le polypeptide est un polypeptide fusionné en phase avec au moins un second polypeptide. Bien que la fusion puisse avoir lieu à un endroit quelconque du premier polypeptide, les extrémités N ou C-terminales sont préférées et notamment l'extrémité N-terminale. Une fusion des activités CDase et UPRTase permet d'améliorer la sensibilité des cellules cibles à la 5-FC et à la 5-FU.

L'homme du métier est capable de cloner les séquences de CDase ou UPRTase à partir des données publiées, de procéder à d'éventuelles mutations, de tester les activités enzymatiques des formes mutantes dans un système acellulaire ou cellulaire selon la technologie de l'art ou en suivant le protocole indiqué dans la demande WO9954481 et de fusionner, notamment en phase, les polypeptides d'activité CDase et UPRTase, et par conséquent tout ou partie des gènes correspondants. Des polypeptides hybrides tels que décrits dans les demandes de brevet WO96/16183 et WO9954481 sont incorporées à la demande par référence.

Selon une autre variante, la substance ayant au moins une activité cytotoxique est un facteur protéique anti-angiogénique. L'angiogénèse est le processus responsable de la formation de nouveaux capillaires à partir du réseau vasculaire déjà existant. Ce processus complexe est finement régulé dans les tissus sains par la balance des effets de nombreux facteurs angiogéniques et anti-angiogéniques. Cependant, dans certaines pathologies, et notamment lors de la formation d'une tumeur, ce processus est dérégulé : les facteurs angiogéniques prennent le pas sur les facteurs anti-angiogéniques ce qui permet une vascularisation importante des tumeurs et par voie de conséquence leur développement rapide et / ou l'apparition de métastases. C'est pourquoi, dans le cadre de la présente invention, un facteur anti-angiogénique est considéré comme étant un agent cytotoxique, notamment antitumoral. Parmi les différents facteurs anti-angiogéniques connus à l'heure actuelle on peut notamment citer l'angiostatine, l'endostatine, le facteur plaquettaire PF4, la thrombospondine-1, le PRP (pour Proliferin Related Protein), le VEGI (pour Vascular Endothelial Growth Inhibitor) les metalloprotéases et l'urokinase.

Selon une autre variante de l'invention, la substance ayant au moins une activité cytotoxique peut être un polypeptide présentant une activité d'activation de l'apoptose cellulaire, et plus particulièrement la protéine p53. La p53 est une phosphoprotéine nucléaire intervenant notamment dans le contrôle de l'expression des protéines impliquées dans le cycle cellulaire (Ozbun et al, 1995, Adv. Cancer Res. 66, 71-141- Selter et al, 1994, Int. J. Biochem. 26, 145-154) et participant à de nombreux processus cellulaires liés à la stabilité du génome et à l'apoptose cellulaire (Harris et al, 1996, J. Natl. Cancer Inst. 88, 1442-1445 ; Kastan et al, 1991, Cancer Res. 51, 6304-6311 ; Kuerbitz et al, 1992, PNAS, 89, 7491-7495). Le gène p53 a été identifié et séquencé. La séquence du cDNA est décrite dans Matlashewski et al. (1984, EMBO J., 3, 3257-3262) et celle de la protéine dans Lamp (1986, Mol. Cell Biol., 6, 1379-1385). De même, des variants polymorphiques naturels et fonctionnels ont été identifiés pour lesquels certains amino acides sont remplacés par d'autres sans toutefois affecter la fonction p53. Par ailleurs, de nombreuses mutations ont été décrites dans la littérature relative au cancer qui peuvent se traduire par une perte de la fonction de cette protéine (Holstein et al, 1991, Science, 253, 49-53 ; Levine et al, 1991, Nature, 351, 453-456). Par exemple, Baker et al, (1989, Science, 244, 217) ont constaté que dans plus de 70% des tumeurs colorectales la fonction de ce gène p53 est perdue. Dans le cadre de la présente invention, il est possible d'utiliser en l'intégralité de la séquence d'acide nucléique codant pour le polypeptide p53 ou une partie seulement de ce polypeptide, ou un polypeptide dérivé ou muté, dans la mesure où la fonction de p53 est conservée. De telles séquences sont bien connues de l'homme du métier et il est possible de se référer par exemple à Matlashewski et al. (1984, EMBO J., 3, 3257-3262), Prives et al. (1994, Cell, 78, 543-546) ou Chen et al. (1996, Gene et Deve., 10, 2438-2451), dont les contenus sont incorporés dans la présente demande. Etant donné les propriétés du polypeptide p53 comme transactivateur transcriptionnel (Farmer et al., 1992, Nature, 358, 83-86) ou comme polypeptide capable d'interagir avec d'autres protéines (Harris, 1996, Carcinogenesis, 17, 1187-1198), l'activité p53 peut être mesurée par l'analyse de l'arrêt du cycle cellulaire en phase G1/S et G2/M, de l'induction de l'apoptose, de la suppression de la transformation cellulaire induite par les oncogènes ou de l'inhibition de l'angiogénèse.

Selon un mode particulier de l'invention, ledit produit de combinaison comporte en outre des quantités acceptables d'un point de vue pharmaceutique d'une prodrogue capable d'être transformée en molécule cytotoxique par une substance ayant au moins une activité cytotoxique. Une telle prodrogue sera notamment sélectionnée dans le groupe consistant en l'acyclovir ou le ganciclovir (GCV), la cyclophosphophamide, la 6-methylpurine deoxyribonucleoside, la 6-thioxanthine, la cytosine ou un de ses dérivés ou l'uracile ou un de ses dérivés. De plus, lorsque ladite prodrogue est la 5-fluorocytosine (5FC) ou la 5-fluorouracile (5-FU ), ledit produit de combinaison peut également comprendre une ou plusieurs substances potentialisant l'effet cytotoxique du 5-FU. On peut citer en particulier, les drogues inhibant les enzymes de la voie de biosynthèse de novo des pyrimidines (par exemple celles citées ci-après), les drogues telles que la Leucovorin (Waxman et al., 1982, Eur. J. Cancer Clin. Oncol. 18, 685-692) qui en présence du produit du métabolisme du 5-FU (5-FdUMP) augmente l'inhibition de la thymidylate synthase ce qui entraîne une diminution du pool de dTMP nécessaire à la réplication et enfin les drogues telles que le méthotréxate (Cadman et al., 1979, Science 250, 1135-1137) qui en inhibant la dihydrofolate réductase et en élevant le pool d'incorporation de PRPP (phosphoribosylpyrophosphate) provoque l'augmentation de 5-FU dans l'ARN cellulaire.

Par « acide nucléique », on entend désigner un fragment d'ADN et/ou d'ARN, double brin ou simple brin, linéaire ou circulaire, naturel isolé ou de synthèse, désignant un enchaînement précis de nucléotides, modifiés ou non, permettant de définir un fragment ou une région d'un acide nucléique sans limitation de taille.

Selon un mode de réalisation préféré de l'invention, l'acide nucléique selon l'invention sera un vecteur. De façon préférée, selon la présente invention, « acide nucléique » désigne un vecteur recombinant d'origine plasmidique ou virale. Le choix des plasmides utilisables dans le cadre de la présente invention est vaste. Il peut s'agir de vecteurs de clonage et/ou d'expression. D'une manière générale, ils sont connus de l'homme du métier et nombre d'entre eux sont disponibles commercialement, mais il est également possible de les construire ou les modifier par les techniques de manipulation génétique. On peut citer à titre d'exemples les plasmides dérivés de pBR322 (Gibco BRL), pUC (Gibco BRL), pBluescript (Stratagène), pREP4, pCEP4 (Invitrogene) ou encore p Poly (Lathe et al., 1987, Gene *57,* 193-201). De préférence, un plasmide mis en oeuvre dans le cadre de la présente invention contient une origine de réplication assurant l'initiation de la réplication dans une cellule productrice et/ou une cellule hôte (par exemple, on retiendra l'origine ColE1 pour un plasmide destiné à être produit dans *E. coli* et le système oriP/EBNA1 si l'on désire qu'il soit autoréplicatif dans une cellule hôte mammifère, (Lupton et Levine, 1985, Mol. Cell. Biol. 5, 2533-2542 ; Yates et al., Nature *313*, 812-815). Il peut en outre comprendre un gène de sélection permettant de sélectionner ou identifier les cellules transfectées (par exemple complémentation d'une mutation d'auxotrophie, gène codant pour la résistance à un antibiotique). Bien entendu, il peut comprendre des éléments supplémentaires améliorant son maintien et/ou sa stabilité dans une cellule donnée (séquence *cer* qui favorise le maintien sous forme de monomère d'un plasmide (Summers et Sherrat, 1984, Cell 36, 1097-1103), séquences d'intégration dans le génome cellulaire .

S'agissant d'un vecteur viral, on peut envisager un vecteur dérivant d'un poxvirus (par exemple virus de la vaccine, notamment MVA, canaripox), d'un adénovirus, d'un rétrovirus, d'un virus de l'herpès, d'un alphavirus, d'un foamy virus ou d'un virus associé à l'adénovirus. On aura de préférence recours à un vecteur non réplicatif et non intégratif. A cet égard, les vecteurs adénoviraux conviennent tout particulièrement à la mise en oeuvre de la présente invention. Toutefois, il convient de noter ici que dans le cadre de la mise en oeuvre de la présente invention, la nature du vecteur revêt peu d'importance.

Les rétrovirus ont la propriété d'infecter et de s'intégrer majoritairement dans les cellules en division et à cet égard sont particulièrement appropriés pour l'application cancer. Un rétrovirus recombinant selon l'invention comporte généralement les séquences LTR, une région d'encapsidation et la séquence nucléotidique selon l'invention placée sous le contrôle du LTR rétroviral ou d'un promoteur interne tels que ceux décrits ci-après. Il peut dériver d'un rétrovirus d'une origine quelconque (murin, primate, félin, humain, etc.) et en particulier du MoMuLV (Moloney murine leukemia virus), MVS (Murine sarcoma virus) ou Friend murine retrovirus (Fb29). Il est propagé dans une lignée d'encapsidation capable de fournir *en trans* les polypeptides viraux gag, pol et/ou env nécessaires à la constitution d'une particule virale. De telles lignées sont décrites dans la littérature (PA317, Psi CRIP GP + Am-12 etc...). Le vecteur rétroviral selon l'invention peut comporter des modifications notamment au niveau des LTR (remplacement de la région promotrice par un promoteur eucaryote) ou de la région d'encapsidation (remplacement par une région d'encapsidation hétérologue, par exemple de type VL30) (voir les demandes WO9601324 et FR2762615).

On pourra également avoir recours à un vecteur adénoviral défectif pour la réplication, c'est à dire dépourvu de tout ou partie d'au moins une région essentielle à la réplication sélectionnée parmi les régions E1, E2, E4 et/ou L1-L5. Une délétion de la région E1 est préférée. Mais elle peut être combinée à d'autres modification(s) / délétion(s) touchant notamment tout ou partie des régions E2, E4 et/ou L1-L5, dans la mesure où les fonctions essentielles défectives sont complémentées *en trans* au moyen d'une lignée de complémentation et/ou d'un virus auxiliaire afin d'assurer la production des particules virales d'intérêt. A cet égard, on peut avoir recours aux vecteurs de l'art antérieur tels que par exemple ceux décrits dans les demandes internationales WO 94/28152 et WO 97/04119. A titre illustratif, la délétion de la majorité de la région E1 et de l'unité de transcription E4 est tout particulièrement avantageuse. Dans le but d'augmenter les capacités de clonage, le vecteur adénoviral peut en outre être dépourvu de tout ou partie de la région E3 non essentielle. Selon une autre alternative, on peut mettre en oeuvre un vecteur adénoviral minimal retenant seulement les séquences essentielles à l'encapsidation, à savoir les ITRs (Inverted Terminal Repeat) 5' et 3' et la région d'encapsidation. Par ailleurs, l'origine du vecteur adénoviral selon l'invention, peut être variée aussi bien du point de vue de l'espèce que du sérotype. Il peut dériver du génome d'un adénovirus d'origine humaine ou animale (par exemple canine, aviaire, bovine, murine, ovine, porcine, simienne) ou encore d'un hybride comprenant des fragments de génome adénoviral d'au moins deux origines différentes. On peut citer plus particulièrement les adénovirus CAV-1 ou CAV-2 d'origine canine, DAV d'origine aviaire ou encore Bad de type 3 d'origine bovine (Zakharchuk et al., Arch. Virol., 1993, 128: 171-176 ; Spibey et Cavanagh, J. Gen. Virol., 1989, 70: 165-172 ; Jouvenne et al., Gene, 1987, 60: 21-28 ; Mittal et al., J. Gen. Virol., 1995, 76: 93-102). Cependant, on préférera un vecteur adénoviral d'origine humaine dérivant de préférence d'un adénovirus de sérotype C, notamment de type 2 ou 5. Un vecteur adénoviral selon la présente invention peut être généré *in vitro* dans Escherichia coli (E. coli) par ligation ou recombinaison homologue (voir par exemple la demande internationale WO 96/17070) ou encore par recombinaison dans une lignée de complémentation. Les différents vecteurs adénoviraux ainsi que leurs techniques de préparation sont connus (voir par exemple Graham et Prevect, 1991, in Methods in Molecular Biology, vol 7, p 109-128 ; Ed : E.J. Murey, The Human Press Inc).

Dans le cas où l'acide nucléique comprend au moins une séquence codant un oligonucléotide et/ou un polypeptide, il convient de préciser que ledit acide nucléique comporte en outre les éléments nécessaires afin d'assurer l'expression de ladite séquence après transfert dans une cellule cible, notamment des séquences promotrices et/ou des séquences de régulation efficaces dans ladite cellule, et éventuellement les séquences requises pour permettre l'excrétion ou l'expression à la surface des cellules cibles dudit polypeptide. Les éléments nécessaires à l'expression sont constitués par l'ensemble des éléments permettant la transcription de la séquence nucléotidique en ARN et la traduction de l'ARNm en polypeptide, notamment les séquences promotrices et/ou des séquences de régulation efficaces dans ladite cellule, et éventuellement les séquences requises pour permettre l'excrétion ou l'expression à la surface des cellules cibles dudit polypeptide. Ces éléments peuvent être régulables ou constitutifs. Bien entendu, le promoteur est adapté au vecteur retenu et à la cellule hôte. On peut citer, à titre d'exemples, les promoteurs eucaryotes des gènes PGK (Phospho Glycérate Kinase), MT (metallothioneine ; Mc Ivor et al., 1987, Mol. Cell Biol., 7, 838-848), α-1 antitrypsine, CFTR, les promoteurs du gène codant pour la créatine kinase musculaire, pour l'actine, pour le surfactant pulmonaire, pour les immunoglobulines, pour la β-actine (Tabin et al., 1982, Mol. Cell Biol., 2, 426-436), SRα (Takebe et al., 1988, Mol. Cell. Biol., 8, 466-472), le promoteur précoce du virus SV40 (Simian Virus), le LTR du RSV (Rous Sarcoma Virus), le promoteur de MPSV, le promoteur TK-HSV-1, le promoteur précoce du virus CMV (Cytomegalovirus), les promoteurs du virus de la vaccine p7.5K pH5R, pK1L, p28, p11 et les promoteurs adénoviraux E1A et MLP ou une combinaison desdits promoteurs. Il peut également s'agir d'un promoteur stimulant l'expression du gène dans une cellule tumorale. On peut citer notamment les promoteurs des gènes MUC-1 surexprimé dans les cancers du sein et de la prostate (Chen et al., 1995, J. Clin. Invest., 96, 2775-2782), CEA (pour carcinoma embryonic antigen) surexprimé dans les cancers du colon (Schrewe et al., 1990, Mol. Cell. Biol., 10, 2738-2748), tyrosinose surexprimé dans les mélanomes (Vile et al., 1993, Cancer Res. 53, 3860-3864), ERB-2 surexprimé dans les cancers du sein et du pancréas (Harris et al., 1994, Gene Therapy, 1, 170-175) et α-fétoprotéine surexprimée dans les cancers du foie (Kanai et al., 1997, Cancer Res., 57, 461-465). Le promoteur précoce du Cytomégalovirus (CMV) est tout particulièrement préféré. Il est également possible d'utiliser une région promotrice tissu-spécifique, notamment lorsque la tumeur à traiter est issue d'un type cellulaire particulier, ou activable dans des conditions définies. La littérature procure un grand nombre d'informations relatives à de telles séquences promotrices. De même, dans ce type de construction d'acide nucléique, il est possible d'introduire des séquences nucléiques « neutres » ou introns qui ne nuisent pas la transcription et sont épissées avant l'étape de traduction. De telles séquences et leurs utilisations sont décrites dans la littérature (WO 94/29471). Ledit acide nucléique pourra également renfermer des séquences requises pour le transport intracellulaire, pour la réplication et/ou pour l'intégration, pour la sécrétion, pour la transcription ou la traduction. De telles séquences sont bien connues de l'homme du métier. Par ailleurs, les acides nucléiques utilisables selon la présente invention peuvent également être des acides nucléiques modifiés de sorte qu'il ne leur est pas possible de s'intégrer dans le génome de la cellule cible ou des acides nucléiques stabilisés à l'aide d'agents, tels que par exemple la spermine, qui en tant que tels n'ont pas d'effet sur l'efficacité de la transfection.

Selon une variante préférée de l'invention, le produit de combinaison selon l'invention comprend au moins un acide nucléique comprenant au moins une séquence codant pour une substance capable d'inhiber l'activité de CSF-1 et au moins une séquence codant pour une substance ayant au moins une activité cytotoxique.

Dans le cadre de la présente invention, il est possible d'utiliser l'intégralité ou une partie seulement de la séquence d'acide nucléique codant pour une substance capable d'inhiber l'activité de CSF-1 ou pour une substance ayant au moins une activité cytotoxique, ou une substance dérivée ou mutée, dans la mesure où les fonctions et les propriétés de ces substances sont conservées. Au sens de la présente invention, on entend par mutation, une délétion et / ou une substitution et / ou une addition d'un ou plusieurs nucléotides. De même, il est envisageable d'utiliser une séquence codant pour un polypeptide hybride provenant de la fusion de la séquence codant pour une substance capable d'inhiber l'activité de CSF-1 ou pour une substance ayant au moins une activité cytotoxique et de la séquence codant pour un polypeptide d'un autre type (par exemple, d'ancrage membranaire, de sécrétion).

Les acides nucléiques et les oligonucléotides selon l'invention, peuvent avantageusement être associés à au moins une substance qui s'associe aux acides nucléiques.

Par « substance qui s'associe aux acides nucléiques », on entend désigner une substance, ou une combinaison de plusieurs substances, qui permet en particulier d'améliorer l'efficacité transfectionnelle et/ou la stabilité d'un vecteur et ou d'un oligonucléotide, particulièrement d'un vecteur d'origine plasmidique, et/ou la protection dudit vecteur ou dudit oligonucléotide *in vivo* à l'égard du système immunitaire de l'organisme hôte (Rolland A, Critical reviews in Therapeutic Drug Carrier System, 15, (1998), 143-198). Ces substances s'associent aux acides nucléiques par interaction électrostatique, hydrophobe, cationique, covalente ou préférentiellement non covalente. De telles substances sont largement documentées dans la littérature accessible à l'homme du métier (voir par exemple Felgner et al., 1987, Proc. West. Pharmacol. Soc. 32, 115-121 ; Hodgson et Solaiman , 1996, Nature Biotechnology 14, 339-342 ; Remy et al., 1994, Bioconjugate Chemistry 5, 647-654). A titre illustratif, mais non limitatif, il peut s'agir de polymères cationiques, de lipides cationiques, mais également de liposomes, de protéines nucléaires ou virales ou encore de lipides neutres, zwitterioniques ou chargés négativement. Ces substances peuvent être utilisées seules ou en combinaison. Des exemples de tels composés, ainsi que de méthodes permettant de mesurer leur capacité à améliorer l'efficacité transfectionnelle et/ou la stabilité d'un vecteur donné, sont notamment disponibles dans les demandes de brevet WO 98/08489, WO 98/17693, WO 98/34910, WO 98/37916, WO 98/53853, EP 890362 ou WO 99/05183. Il peut notamment s'agir de substances lipidiques telles que le DOTMA (Felgner et al., 1987, PNAS, 84, 7413-7417), le DOGS ou Transfectam™ (Behr et al., 1989, PNAS, 86, 6982-6986), de la DMRIE ou DORIE (Felgner et al., 1993, Methods, 5, 67-75), du DC-CHOL (Gao et Huang, 1991, BBRC, 179, 280-285), du DOTAP™ (McLachlan et al., 1995, Gene Therapy, 2, 674-622) ou de la Lipofectamine™.

Avantageusement, ces lipides cationiques sont sélectionnés parmi les lipides cationiques de formule (voir EP 901 463) : 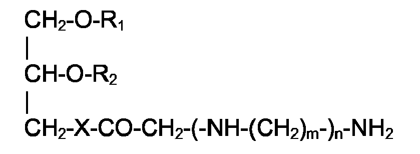 dans laquelle :
R₁, R₂, identiques ou différents, sont des alkyles C₆-C₂₃ ou des alcényles C₆-C₂₃, linéaires ou ramifiés, ou des carbonyles d'alkyles C₆-C₂₃ ou des carbonyles d'alcényles C₆₋C₂₃, linéaires ou ramifiés,
X est O, S, S(O) ou -NR₃, R₃ sont des atomes d'hydrogène ou des C₁-C₄ alkyles,
n est un nombre entier positif compris entre 1 et 6,
m est un nombre entier positif compris entre 1 et 6, et quand n > 1, m peut varier à l'intérieur d'une même molécule.

La substance qui s'associe aux acides nucléiques peut également être un polymère cationique tel que par exemple la polyamidoamine (Haensler et Szoka, Bioconjugate Chem. 4 (1993), 372-379), un polymère "dendrimer" (WO 95/24221), du polyéthylène imine ou du polypropylène imine (WO 96/02655), du chitosan, un poly(aminoacide) comme la polylysine (US- 5,595,897 or FR- 2 719 316) ; un composé polyquaternaire ; la protamine; les polyimines; le polyéthylène imine ou le polypropylène imine (WO 96/02655); les polyvinylamines; les polymères polycationiques substitués par le DEAE, comme les pullulanes, les celluloses; la polyvinylpyridine; les polymethacrylates; les polyacrylates; les polyoxéthanes; le polythiodiethylaminomethylethylene (P(TDAE)); la polyhistidine; la polyornithine, le poly-p-aminostyrène; les polyoxéthanes; les co-polymethacrylates (par exemple les copolymères d'HPMA; N-(2-hydroxypropyl)-methacrylamide); les composés décrits dans US-A-3,910,862, les complexes de polyvinylpyrrolide de la DEAE avec le méthacrylate, le dextran, l'acrylamide, les polyimines, l'albumine, le 1-dimethylaminomethylmethacrylate et le chlorure d'ammonium de polyvinylpyrrolidonemethylacrylaminopropyltrimethyl; les polyamidoamines; les composés télomériques (demande de brevet EP0965583). Néanmoins, cette liste n'est pas exhaustive et d'autres polymères cationiques connus peuvent être utilisés pour obtenir les complexes d'acides nucléiques de l'invention. De plus ces lipides et polymères cationiques peuvent être fluorinés (voir par exemple WO 98/34910).

La substance qui s'associe aux acides nucléiques peut également être un lipide neutre, zwitterionique ou chargé négativement. Ces lipides neutres, zwitterioniques ou chargés négativement peuvent être, par exemple, sélectionnés dans le groupe comprenant les phospholipides naturels d'origine animale ou végétale, tels que la phosphatidylcholine, la phosphocholine, la phosphatidyléthanolamine, la sphingomyéline, la phosphatidylsérine, le phosphatidylinositol , la céramide ou la cérebroside et leurs analogues ; les phospholipides synthétiques qui comportent généralement, mais non exclusivement deux chaînes d'acide gras identiques, tels que la dimyristoylphosphatidylcholine, la dioleoylphosphatidylcholine, la dipalmitoylphosphatidylcholine, la distearoylphosphatidylcholine, la phosphatidyléthanolamine (PE) et le phosphatidylglycérol, et leurs analogues ; la phosphatidylcholine, la cardiolipine, la phosphatidyléthanolamine, mono-, di- ou triacylglycérol, et l'alpha-tocophérol et leurs analogues ; le phosphatidylglycérol, l'acide phosphatidique ou l'analogue d'un phospholipide similaire ; le cholestérol, les glycolipides, les acides gras, les sphingolipides, les prostaglandines, les gangliosides, les niosomes, ou tout autre amphiphile naturel ou synthétique.

Par ailleurs, les oligonucléotides et les acides nucléiques mis en oeuvre dans le cadre de la présente invention peuvent en outre comprendre des éléments de ciblage pouvant permettre de diriger le transfert desdite(s) séquence(s) d'acide nucléique vers certains types cellulaires ou certains tissus particuliers (cellules tumorales, cellules de l'épithélium pulmonaire, cellule hématopoïétique, cellule musculaire, cellule nerveuse...). Ils peuvent également permettre de diriger le transfert d'un oligonucléotide ou d'un acide nucléique vers certains compartiments intracellulaires préférés tels que le noyau et les mitochondries. Il peut en outre s'agir d'éléments facilitant la pénétration à l'intérieur de la cellule ou la lyse des endosomes. De tels éléments de ciblage sont largement décrits dans la littérature. Il peut par exemple s'agir de tout ou partie de lectines, de peptides, notamment le peptide JTS-1 (voir demande de brevet WO 94/40958), d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogèniques, de peptides de localisation nucléaire, ou d'une combinaison de tels composés. En particulier, il peut s'agir de résidus galactosyl permettant de cibler le récepteur des asialoglycoprotéines à la surface des cellules hépatiques, de ligands pouvant interagir avec des récepteurs tels que des récepteurs de facteurs de croissance, des récepteur de cytokines, de lectines, de protéines d'adhésion, il peut également s'agir d'un fragment d'anticorps tel que le fragment Fab, d'un peptide fusogénique INF-7 dérivé de la sous unité HA-2 de l'hémagglutinine du virus influenza (Plank et al., 1994, J. Biol. Chem. 269, 12918-12924), d'un signal de localisation nucléaire dérivé de l'antigène T du virus SV40 ou de la protéine EBNA-1 du virus Epstein Barr.

L'invention concerne en outre une composition pharmaceutique caractérisée en ce qu'elle comprend un oligonucléotide, capable d'inhiber l'expression de CSF-1, selon l'invention et/ou un acide nucléique comprenant une séquence codant pour un tel oligonucléotide et un véhicule acceptable d'un point de vue pharmaceutique. L'invention concerne également une composition pharmaceutique caractérisée en ce qu'elle comprend un produit de combinaison tel que décrit précédemment et un véhicule acceptable d'un point de vue pharmaceutique. Un tel support est préférentiellement isotonique, hypotonique ou faiblement hypertonique et présente une force ionique relativement faible, tel que par exemple une solution de sucrose. Par ailleurs, un tel support peut renfermer tout solvant, ou liquide aqueux ou partiellement aqueux tel que de l'eau stérile non pyrogène. Le pH de la formulation est en outre ajusté et tamponné afin de répondre aux exigences d'utilisation *in vivo*. La formulation peut également inclure un diluant, un adjuvant ou un excipient acceptable d'un point de vue pharmaceutique, de même que des agents de solubilisation, de stabilisation, de préservation. Pour une administration injectable, on préfère une formulation en solution aqueuse, non-aqueuse ou isotonique. Elle peut être présentée en dose unique ou en multidoses, sous forme liquide ou sèche (poudre, lyophilisat...) susceptible d'être reconstituée de manière extemporanée par un diluant approprié.

L'invention concerne également une méthode pour inhiber l'expression de CSF-1 consistant à mettre en contact une cellule qui exprime CSF-1 avec un oligonucléotide selon l'invention et/ou avec un acide nucléique comprenant une séquence codant pour un tel oligonucléotide.

Un autre objet selon l'invention consiste en l'utilisation d'un oligonucléotide, capable d'inhiber l'expression de CSF-1, ou d'un acide nucléique comprenant une séquence codant pour une tel oligonucléotide pour la préparation d'un médicament destiné au traitement du corps humain ou animal, destiné notamment au traitement du cancer.

L'invention consiste également en l'utilisation d'une substance capable d'inhiber l'activité de CSF-1 pour la préparation d'un médicament pour améliorer l'efficacité d'un traitement antitumoral.

Les dosages les mieux appropriés varient en fonction de différents paramètres tels que par exemple l'individu ou la maladie à traiter ou la voie d'administration, ou encore de l'organe/tissus hôte. A titre indicatif on peut citer des doses d'oligonucléotides allant de 0.6 à 6.5 mg/kg/jour

Un autre objet selon l'invention consiste en l'utilisation d'un produit de combinaison tel que décrit ci-dessus, pour la préparation d'un médicament destiné au traitement du corps humain ou animal, destiné notamment au traitement du cancer.

Le produit de combinaison selon l'invention peut être mis en oeuvre dans le cadre d'une utilisation simultanée, consécutive ou étalée dans le temps. Simultanément fait référence à une co-administration. Dans ce cas, les composés (i) et (ii) peuvent être mélangées préalablement à l'administration ou peuvent être administrées en même temps dans la cellule ou l'organisme hôte. Il est également possible de les administrer consécutivement, c'est à dire l'une après l'autre, quelle que soit la composante du produit de combinaison selon l'invention administrée en premier. Enfin, on peut avoir recours à un mode d'administration étalé dans le temps ou intermittent qui s'arrête et reprend à intervalle régulier ou non de l'un et/ou de l'autre des deux composantes. On indique que les voies et les sites d'administration des deux composantes peuvent être différents.

Selon un mode de réalisation préféré de l'invention, la substance capable d'inhiber l'activité de CSF-1 et/ou l'acide nucléique, comprenant au moins une séquence codant pour une substance capable d'inhiber l'activité de CSF-1 est administrée préalablement de façon à ce que la concentration en CSF-1 dans le plasma de l'organisme hôte soit inférieure à 350 pg/ml au moment de l'administration de la substance ayant au moins une activité cytotoxique.

La quantité de produit de combinaison à administrer varie en fonction de différents paramètres tels que par exemple la nature de la substance capable d'inhiber l'activité de CSF-1, la nature de la substance ayant au moins une activité cytotoxique, l'individu ou la maladie à traiter ou la voie d'administration, ou encore de l'organe/tissus hôte. A titre indicatif, on peut citer des doses de substances ayant au moins une activité cytotoxique de 75 mg/kg/j de CDDP en une fois et de 500 mg/kg/j pendant 5 jours dans le cas du cancer du col utérin.

Selon l'invention, les voies d'administration envisageables sont multiples. On peut citer par exemple la voie systémique, intragastrique, sous-cutanée, intracardiaque, intramusculaire, intraveineuse, intralymphatique, intrapéritonéale, intratumorale, intranasale, intrapulmonaire ou intratrachéale. Pour ces trois derniers modes de réalisation, une administration par aérosol ou instillation est avantageuse. De manière tout à fait préférée, l'administration de l'oligonucléotide ou du produit de combinaison selon l'invention est réalisée par voie intralymphatique intratumorale ou peritumorale, c'est à dire dans une tumeur accessible, à sa périphérie ou dans un vaisseau lymphatique ou un ganglion relié à l'organe affecté ou à la tumeur.

L'invention s'étend également à une méthode pour le traitement des maladies par thérapie génique, caractérisée en ce que l'on administre à un organisme ou à une cellule hôte ayant besoin d'un tel traitement un produit de combinaison selon l'invention comprenant au moins un acide nucléique et/ou au moins un oligonucléotide.

Avantageusement, dans le cas d'un traitement des maladies par thérapie génique, l'acide nucléique, en fonction de la nature du (ou des) vecteur(s) utilisé(s), comprendra dans sa forme prête à être administrer :
- lorsque le vecteur est d'origine plasmidique, de 0,01 à 100 mg d'ADN, de préférence entre 0,05 à 10 mg et, de manière tout à fait préférée de 0,5 à 5 mg ;
- lorsque le vecteur est d'origine virale, entre 10⁴ et 10¹⁴ ufp (unités formant des plages), avantageusement entre 10⁵ et 10¹³ ufp et de préférence entre 10⁶ et 10¹² ufp.

Lorsque ledit traitement met en oeuvre une substance ayant une activité UPRTase, il peut être avantageux d'administrer en outre une seconde substance présentant une activité CDase. Dans ce dernier cas, l'administration des séquences UPRTase et CDase peut être simultanée ou consécutive, l'ordre d'administration étant sans importance. Selon un tel mode de réalisation, la méthode de traitement comprendra également une étape supplémentaire selon laquelle on administrera à l'organisme ou la cellule hôte des quantités acceptables d'un point de vue pharmaceutique d'une prédrogue, avantageusement d'un analogue de cytosine et, en particulier de la 5-FC. A titre illustratif, une dose de 50 à 500 mg/kg/jour peut être employée avec une préférence pour 200 mg/kg/jour. Cette prédrogue peut être administrée selon les pratiques standards et ceci de manière préalable, concomittante ou encore postérieure à celle du produit de combinaison de la présente invention. La voie orale est préférée. On peut administrer une dose unique de prédrogue ou des doses répétées pendant un temps suffisamment long pour permettre la production du métabolite toxique au sein de l'organisme ou de la cellule hôte.

Parmi les applications envisageables, on peut citer les cancers du sein, de l'ovaire, de l'utérus (notamment ceux induits par les papillomas virus), de la prostate, du poumon, de la vessie, du foie, du colon, du pancréas, de l'estomac, de l'oesophage, du larynx du système nerveux central et du sang (lymphomes, leucémie etc...) des os (sarcomes).

Selon un mode avantageux de l'invention, l'utilisation ou la méthode de traitement de l'invention est associée à un traitement antérieur ou ultérieur du patient par chirurgie (notamment par ablation de la tumeur partiellement ou totalement) ou par radiothérapie. Dans ce cas particulier, le traitement selon l'invention est appliqué de manière préalable, concomitante ou fait suite audit second traitement. De manière préférée, ce traitement sera appliqué suite audit second traitement.

De façon similaire, l'efficacité du transfert intra-cellulaire de l'oligonucléotide ou de l'acide nucléique peut être avantageusement facilitée par exemple par combinaison avec un traitement d'électroporation (Vicat et al., 2000, Human Gene Therapy, 11, 909-916) et/ou un traitement visant à modifier la perméabilité des vaisseaux sanguins dans lesquels est réalisée l'administration (WO 98/58542), ou par tout autre moyen décrit dans la littérature.

Les exemples qui suivent ont pour but d'illustrer les différents objets de la présente invention et n'ont par conséquence aucun caractère limitatif.

### EXEMPLE 1.

### Oligonucléotides:

Tous les oligonucléotides utilisés dans cette étude (SEQ ID NO:2 à SEQ ID NO:5) ont été synthétisés par Eurogentec (Seraing, Belgique) et purifiés par chromatographie. Les modifications des oligonucléotides utilisés dans cette étude sont de deux ordres. Premièrement, une modification du squelette phosphodiester en phosphorothioate. Deuxièmement, une modification de la chimie des bases, l'uridine (U) étant remplacée par une 5-(1-propynyl)-2'deoxyuridine (pdU) et la cytosine C par une 5-(1-propynyl)-2'-deoxycytidine (pdC). Le taux de substitution des bases a été limité à 50 % du contenu en C et U.

La capacité des oligonucléotides utilisés à s'hybrider à SEQ ID NO:1 a été vérifiée, *in vitro,* par des expériences de dénaturation thermique. La formation du duplex oligonucléotide/SEQ ID NO:1 a été suivie en mesurant l'absorbance à 260nm d'une solution contenant 1 µm d'oligonucléotide et 1 µm de SEQ ID NO:1, en fonction de la température. Toutes les expériences ont été réalisées dans un tampon 10 mM cacodylate, 50mM NaCl, pH 7,0.

### Culture cellulaire et transfection des oligonucléotides.

La lignée NS2T2A1 est une lignée de cellules de sein humaine (Ma et al., Int. J. Cancer ,1998, 78:112-119) obtenue par mammoplastie, transformées par SV40. Cette lignée sécrète le CSF-1 de manière linéaire en fonction de la croissance cellulaire.

Les cellules ont été cultivées dans du milieu M5 (Gibco, France) complémenté avec 5 % de sérum de cheval comme décrit par Ma, et al. (Int. J. Cancer ,1998, 78:112-119) L'ensemencement a été fait dans des plaques de 6 puits à une concentration de 5.10⁵ cellules/ml/puit. La transfection par les oligonucléotides a été réalisée 24h après lorsque les cellules étaient en phase de croissance exponentielle et qu'elles avaient atteint 60 % à 80 % de confluence.

La transfection par les oligonucléotides a été réalisée à l'aide de cytofectine GS-3815 (Glen reaserch, Sterling, VA). Les oligonucléotides et la cytofectine ont été dilués séparément dans des tubes en polystyrène avec du milieu opti-MEM (Gibco, France), puis la cytofectine est mélangée avec un volume égal d'oligonucléotide dilué. Le mélange est laissé 15 minutes à température ambiante de manière à permettre la formation des complexes oligonucléotides/cytofectine. Ensuite, du milieu M5 est ajouté à la solution de manière à obtenir les concentrations finales suivantes : 2,4 µg/ml de cytofectine et 250 nM d'oligonucléotides. Toutes les transfections ont été réalisées dans du milieu M5 et dans un volume final de 1 ml. Les complexes oligonucléotides/cytofectine ont été laissés en contact avec les cellules pendant 24h. Toutes les étapes décrites ci-dessus ont été réalisées en conditions stériles.

Toutes les expériences ont été réalisées en triplicate pour chaque concentration d'oligonucléotides.

### Quantification de CSF-1

La quantité de CSF-1 sécrété dans le milieu de culture des cellules NS2T2A1 a été mesurée, par un test ELISA, 24h après le début de la transfection, en se référant strictement au protocole décrit par le fournisseur (R&D systems, Europe Ltd, UK). Les résultats sont exprimés en pourcentage de CSF-1 exprimé par les cellules transfectées par rapport au CSF-1 exprimé par à une lignée cellulaire non transfectée.

### Résultats

Les résultats obtenus sont regroupés dans le tableau ci-dessous.

| Séquence de l'oligonucléotide | Région d'hybridation sur SEQ ID NO:1 | Pourcentage de CSF-1 exprimé |
|---|---|---|
| SEQ ID NO:2 | 135-152 | 70% |
| SEQ ID NO:3 | 284-301 | 39% |
| SEQ ID NO:4 | 341-358 | 50% |
| SEQ ID NO:5 | 409-426 | 90% |

Ces résultats indiquent clairement que les cellules transfectées par les oligonucléotides selon l'invention expriment moins de CSF-1 que les cellules non-transfectées par lesdits oligonucléotides.

## Revendications

1. Produit de combinaison comprenant :
(i) au moins une substance capable d'inhiber l'activité de CSF-1 et/ou au moins un acide nucléique, comprenant au moins une séquence codant pour une substance capable d'inhiber l'activité de CSF-1 et,
(ii) (ii) au moins une substance ayant au moins une activité cytotoxique et/ou au moins un acide nucléique, comprenant au moins une séquence codant pour une substance ayant au moins une activité cytotoxique.
**caractérisé en ce que** la substance capable d'inhiber l'activité de CSF-1 est un anticorps

2. Produit de combinaison selon la revendication 1 **caractérisé en ce que** la substance ayant au moins une activité cytotoxique est choisie parmi les substances interagissant avec l'ADN, les antimétabolites, les inhibiteurs de topo-isomérases, les agents du fuseau et les agents cytostatiques.

3. Produit de combinaison selon l'une des revendications 1 et 2, **caractérisé en ce que** la substance ayant au moins une activité cytotoxique est un antigène associé aux tumeurs.

4. Produit de combinaison selon l'une des revendications 1 à 3, **caractérisé en ce que** la substance ayant au moins une activité cytotoxique est choisie parmi les cytokines, les polypeptides présentant une activité de chimio-attraction, les protéines codées par un gène suicide, les facteurs protéiques anti-angiogéniques et les polypeptides présentant une activité d'activation de l'apoptose cellulaire.

5. Composition comprenant au moins un produit de combinaison selon l'une des revendications 1 à 4 et un véhicule acceptable d'un point de vue pharmaceutique.

6. Utilisation d'un produit de combinaison selon les revendications 1 à 4, pour la préparation d'un médicament pour le traitement du cancer.

7. Utilisation d'un anticorps capable d'inhiber l'activité de CSF-1 pour la préparation d'un médicament pour améliorer l'efficacité d'un traitement antitumoral.
